# EUROPEAN PATENT SPECIFICATION

(11) **EP 1 765 270 B1**
(45) Date of publication and mention of the grant of the patent: **17.09.2008**
(21) Application number: 05755443.8
(22) Date of filing: 15.06.2005
(51) Int. Cl.: A61Q 9/04, A61K 8/35

(54) **IMPROVEMENTS IN OR RELATING TO DEPILATORY COMPOSITIONS**
VERBESSERUNGEN VON ODER IM ZUSAMMENHANG MIT DEPILATORISCHEN ZUSAMMENSETZUNGEN
AMELIORATIONS APPORTEES OU SE RAPPORTANT A DES COMPOSITIONS EPILATOIRES

(30) Priority: 15.06.2004 GB 0413298
(43) Date of publication of application: 28.03.2007
(73) Proprietor: Reckitt Benckiser (UK) Limited, Slough, Berkshire SL1 3UH (GB)
(72) Inventor: DE LA TORRE, Frederic, Antibes 06600 (FR); WILKINSON, Paul, Andrew, David, Hull HU8 7DS (GB)
(74) Representative: Cawdell, Karen Teresa
(86) International application number: PCT/GB2005/002362
(87) International publication number: WO 2005/123022

(56) References cited:
- EP-A- 0 085 894
- US-A- 3 147 288
- US-A- 4 548 811
- US-A1- 2003 175 333
- US-B1- 6 479 043
- PATENT ABSTRACTS OF JAPAN vol. 1999, no. 06, 31 March 1999 (1999-03-31) & JP 02 048518 A (TEE SHIYAN CHANGU), 19 February 1990 (1990-02-19)

## Description

The present invention relates to depilatory compositions comprising a depilatory compound having a thiol group and to a method of depilation using such compositions.

Compositions for removing superfluous body hair are well known and are of various types. One type of composition requires initial heating before being applied to the skin in a generally molten state. It is then allowed to solidify before being removed from the skin together with unwanted hair.

Another type of depilatory composition is in the form of a composition, such as a cream, which can be applied to the skin, generally at room temperature. The cream includes a substance that degrades hair keratin. After the composition has been applied it is allowed to remain on the skin to degrade the hairs and then removed together with the degraded hairs.

Depilatory compositions of the type which degrades the hairs comprise a depilatory compound which is able to degrade the hairs. Depilatory compounds in common use, such as potassium thioglycolate, and other such compounds having a thiol group, have a disadvantage in that they have an unattractive smell. The composition may intrinsically have an unattractive smell which is noticed by a user when it is removed from a container. During use, however, the unattractive smell can increase due to reaction of the depilatory compound with the hair which is being or which has been degraded. This is particularly unpleasant for the consumer. It would be desirable to have depilatory compositions with a reduced smell, in particular a reduced smell when in use.

Although agents are known to reduce the malodour when added to depilatory compositions, such agents also reduce the efficacy of the depilatory compound. An example of such an agent is zinc oxide.

Furthermore depilatory compositions typically contain compounds which can irritate and even damage the skin. For example they typically contain sodium hydroxide to provide a high pH. The depilatory composition are applied to the skin and allowed to act on the skin for a sufficient time to degrade the hairs. However, the compositions should not be allowed to act on the skin for longer than a certain time so as to reduce the irritant effect and possible damage to the skin. Although instructions are typically provided with depilatory compositions informing the user of the correct residence time, users do not always read them or follow the instructions correctly. It would therefore be desirable to have a composition which has an appropriate end-of-life indication after a suitable residence time so that a user knows when it is appropriate to remove the composition or which indicates when the composition is likely to have remained on the user's skin too long.

The present invention provides a depilatory composition comprising a depilatory compound having a thiol group, and a t-butyl hydroquinone.

We have surprisingly discovered that adding t-butyl hydroquinone to a depilatory composition comprising a depilatory compound having a thiol group may counteract the malodour of the depilatory composition and/or provide an end-of-use indication or indication that the composition should be removed from the skin by providing a colour change to the composition.

The t-butyl hydroquinone compound have surprisingly been found to reduce or remove the bad smell of the depilatory composition without substantially adversely affecting the ability of the depilatory compound to degrade hair. It has also been found that t-butyl hydroquinone may cause the depilatory composition to exhibit a colour change after an appropriate time. For example, depilatory compositions typically are white. However, when a t-butyl hydroquinone is present in the composition it has surprisingly been found that the composition exhibits a colour change, for example turning to yellow and then to brown after about 10 minutes, which is an appropriate residence time on the skin. It is believed, although we are not bound by this theory, that this is caused by an oxidation reaction after the composition is exposed to air. The colour change can be controlled, for example, by altering the concentration of t-butyl hydroquinone or by changing the nature of the compound. Furthermore including a reducing agent in the composition can assist in retarding the development of the colour change, thus enabling the timing of the change to be controlled. Inclusion of compounds, such as an amine or a metal-containing compound, which can react with t-butyl hydroquinone, may give more intensity to the colour.

Suitable metal containing compounds are lithium, potassium, sodium or magnesium in the form of hydroxides or silicates or in hectorite or montmorillonite structures.

The depilatory compound having a thiol group may be any compound capable of degrading keratin. Examples of such compounds are potassium thioglycolate, dithioerythritol, thioglycerol, thioglycol, thioxanthine, thiosalicylic acid, N-acetyl-L-cysteine, lipoic acid, sodium dihydrolipoate 6,8-dithioocatanoate, sodium 6,8-diothioocatanoate, a hydrogen sulphide salt, thioglycolic acid, 2-mercaptopropionic acid, 3-mercaptopropionic acid, thiomalic acid, ammonium thioglycolate, glyceryl monothioglycolate, monoethanolamine thioglycolate, monoethanolamine thioglycolic acid, diammoniumdithiodiglycolate, ammonium thiolactate, monoethanolamine thiolactate, thioglycolamide, homocysteine, cysteine, glutathione, dithiothreitol, dihydrolipoic acid, 1,3-dithiopropanol, thioglycolamide, glycerylmonothioglycolate, thioglycolhydrazine, keratinase, guanidine thioglycolate, calcium thioglycolate and/or cysteamine. A single compound or a mixture of two or more compounds may be used.

Preferably the depilatory compound is potassium thioglycolate.

The depilatory compound is preferably present in the composition in an amount of from 1 to 10 wt% based on the total weight of the composition, more preferably from 2 to 7 wt%.

The composition preferably comprises from 0.001 to 10 wt% of t-butyl hydroquinone based on the total weight of the composition, preferably from 0.01 to 1 wt%, most preferably from 0.05 to 0.1 wt%.

The depilatory composition may, for example, comprise components which accelerate the keratin degradation reaction such as urea thiourea, dithioerythritol, dimethyl isosorbide (DMI), ethoxydiglycol (Transcutol) or methyl propyl diol (MT diol). The composition desirably comprises up to 15 wt% of the accelerator based on the total weight of the composition, preferably from 5 to 15 wt% and more preferably from 7 to 10 wt%.

The composition may comprise a source of alkalinity, for example an alkali metal hydroxide such as sodium hydroxide or potassium hydroxide. Desirably the pH of the composition of the present invention is at least 12, more preferably at least 12.4.

Preferably the alkali metal hydroxide is present in an amount of at least 0.001 mol/100 g of composition, preferably in an amount of at least 0.01 mol/100 g composition.

The depilatory composition of the present invention may comprise water, suitably in an amount of at least 40 or 50 wt% based on the total weight of the composition, more preferably at least 60 wt% and even more preferably at least 68 wt%. Preferably the water is present in an amount of up to 95 wt% based on total weight of composition, more preferably up to 85 wt% and even more preferably up to 75 wt%.

The depilatory composition may, for example, be in the form of an oil-in-water emulsion, a water-in-oil emulsion, a microemulsion, a multiple emulsion, a lotion, cream, gel or foam.

The depilatory composition may comprise further components such as perfumes, oils, pigments, clays, fillers such as lithium sodium magnesium silicate, magnesium trisilicate and titanium dioxide.

If the depilatory composition is in the form of a cream it may, for example, use a conventional cream base, such as a mixture of polypropylene glycol ester and cetostearyl alcohol.

The composition of the present invention may be prepared, for example, by mixing the various components together, preferably at a temperature not exceeding 85°C, and preferably at a temperature of from 65°C to 85°C. More preferably all of the components excluding the depilatory compound are first mixed together at this elevated temperature, the resultant composition is actively or passively cooled and the depilatory compound added to the cooled composition at a temperature of from 15°C to 40°C, preferably at ambient temperature (e.g. about 20°C). The source of alkalinity such as the alkali metal hydroxide may be added at any stage of the process, but preferably after the depilatory compound is added. The t-butyl hydroquinone can also be added at any stage of the process. It is generally added in the form of a solution in a solvent such as glycol, and is preferably added at the same time or with the depilatory compound.

The present invention also provides a method of depilation comprising:
a. applying to the skin a composition as defined in above;
b. allowing the composition a residence time on the skin to degrade hairs;
c. at the end of the residence time removing the composition and depilated hairs from the skin; and
d. rinsing the skin.

Preferably the residence time on the skin is from 3 to 10 minutes. Desirably the appropriate residence time is coordinated with a suitable colour change of the composition such that a user of the composition would know when it is appropriate to remove the composition. The composition may be removed from the skin using a spatula or scraper device. Where the colour change is gradual, the user is preferably provided also with an indicator chart or card showing the colour which the composition should attain before the composition and depilated hairs are removed from the skin.

Hence the present invention provides a method of depilation comprising;
a. providing a composition according to the invention as defined above in a container,
b. applying the composition to the skin such that the composition is exposed to the air,
c. allowing the composition to reside on the skin exposed to air until a colour change of the composition occurs,
d. removing the composition and depilated hairs from the skin until a colour change of the composition has occurred, and
e. rinsing the skin.

Suitably the composition will be provided in a sealed single use ampule or in tube or a bottle with a replaceable cap such that the composition is not exposed to air on storage.

The purpose of the container is to prevent excessive contact between the composition and air prior to exposure on the skin of the consumer.

The present invention further provides the use of t-butyl hydroquinone in a depilatory composition comprising a depilatory compound having a thiol group to reduce the malodour of said depilatory compound when said composition is in use.

The present invention additionally provides the use of a quinone and/or phenol compound in a depilatory composition comprising a depilatory compound having a thiol group to provide a colour change to said composition after said composition has been applied to the skin of a user for a predetermined time.

The use of the accelerant such as a monovalent or divalent hydroxide (for example Ca(OH)₂ or KOH) advantageously results in accelerated hair removal action of the depilatory composition. However, the use of monovalent or divalent hydroxides (in particular Ca(OH)₂ )in the composition results in a more sudden burst of odour which is disadvantageous to the end user of the depilatory composition. Furthermore, the use of such accelerants results in the composition requiring a shorter residence time on the users skin. A problen associated with such compositions requiring a shorter residence time is that the user often leaves the composition on the skin for longer than required, thereby causing irritation.

Accordingly, there is further provided a use of a quinone and/or phenol compound in combination with a monovalent and/or a divalent hydroxide in a depilatory composition comprising a depilatory compound having a thiol group, to provide a colour change to the composition after the composition has been exposed to the air whilst on the skin of a user for a predetermined time.

A suitable hydroxide may be Ca(OH)₂; a typical colour change on the skin of a user when such a hydroxide is used may be from a substantially white colour when the composition is applied to a users skin, through yellow to brown after about 10 minutes; this being the appropriate residence time on the skin.

The hydroxide may also be KOH. A typical colour change when KOH is used would be from substantially white to substantially pink within about a 10 minute timeframe. It is, of course, envisaged that other hydroxides would produce different colour changes, each colour change used being dependent on the hydroxide included.

Preferably, the depilatory composition includes the hydroxide in an amount in the range 1.5% to 8% by weight of the composition. It is particularly preferred that the hydroxide is in the range 2.5% to 6.5% by weight of the composition, further preferably 3.0 to 4.5% by weight.

The present invention is further illustrated in the following Examples:

### Example 1

A depilatory composition comprising 0.1 wt% tert-butyl hydroquinone (TBHQ) was prepared. The composition was as follows:

| **Ingredient ( Trade Name)** | **% w/w** | **500g Lab Sample (g)** |
|---|---|---|
| Ceteareth-20 | 2.2 | 11 |
| Cetearyl alcohol | 5.5 | 27.5 |
| PPG-15 Stearyl Ether | 1.5 | 7.5 |
| | | |
| Deionised Water | 47.4 | 237 |
| Calcium Hydroxide | 2.9 | 14.5 |
| Sodium Gluconate | 0.1 | 0.5 |
| Magnesium Trisilicate | 0.5 | 2.5 |
| Titanium dioxide/propylene glycol paste | 0.6 | 3 |
| | | |
| Deionised Water | 20.5 | 102.5 |
| Urea | 8 | 40 |
| Lithium Magnesium Sodium Silicate | 0.5 | 2.5 |
| Acrylates Copolymer | 0.1 | 0.5 |
| | | |
| Potassium thioglycolate | 10 | 50 |
| Crosilk Liquid ( Silk Extract) | 0.1 | 0.5 |
| TBHQ | 0.1 | 0.5 |
| **TOTALS** | **100%** | **500g** |

The composition was exposed to air following removal from an air-tight container. The initial colour was white. After 2 minutes the composition changed colour to Pantone no. 468 C/55C Matt, after 3 minutes to 7501C/106C Matt, after 5 minutes to 467C/55C Matt and after 10 minutes to beige (no good Pantone match).

A comparative example was prepared without TBHQ (replaced by deionised water as in comparative Example 1 - see below). This also had an initial colour which was white. The comparative example exhibited no colour change on exposure to air, following the experimental procedure as for Example 1.

### Example 2

A depilatory composition comprising 0.05 wt% TBHQ was prepared similarly as in Example 1. The composition was as follows:

| **Ingredient ( Trade Name )** | **% w/w** | **500g Lab Sample (g)** |
|---|---|---|
| Ceteareth-20 | 2.2 | 11 |
| Cetearyl alcohol | 5.5 | 27.5 |
| PPG-15 Stearyl Ether | 1.5 | 7.5 |
| | | |
| Deionised Water | 47.45 | 237.25 |
| Calcium Hydroxide | 2.9 | 14.5 |
| Sodium Gluconate | 0.1 | 0.5 |
| Magnesium Trisilicate | 0.5 | 2.5 |
| Titanium dioxide/propylene glycol paste | 0.6 | 3 |
| | | |
| Deionised Water | 20.5 | 102.5 |
| Urea | 8 | 40 |
| Lithium Magnesium Sodium Silicate | 0.5 | 2.5 |
| Acrylates Copolymer | 0.1 | 0.5 |
| | | |
| Potassium thioglycolate | 10 | 50 |
| Crosilk Liquid ( Silk Extract) | 0.1 | 0.5 |
| TBHQ | 0.05 | 0.25 |
| **TOTALS** | **100%** | **500g** |

### Comparative Example 1

A depilatory composition not containing any quinone or phenol compound was prepared similarly as in Example 1. The composition was as follows:

| **Ingredient ( Trade Name )** | **% w/w** | **500g Lab Sample (g)** |
|---|---|---|
| Ceteareth-20 | 2.2 | 11 |
| Cetearyl alcohol | 5.5 | 27.5 |
| PPG-15 Stearyl Ether | 1.5 | 7.5 |
| | | |
| Deionised Water | 47.5 | 237.5 |
| Calcium Hydroxide | 2.9 | 14.5 |
| Sodium Gluconate | 0.1 | 0.5 |
| Magnesium Trisilicate | 0.5 | 2.5 |
| Titanium dioxide/propylene glycol paste | 0.6 | 3 |
| | | |
| Deionised Water | 20.5 | 102.5 |
| Urea | 8 | 40 |
| Lithium Magnesium Sodium Silicate | 0.5 | 2.5 |
| Acrylates Copolymer | 0.1 | 0.5 |
| | | |
| Potassium thioglycolate | 10 | 50 |
| Crosilk Liquid ( Silk Extract) | 0.1 | 0.5 |
| **TOTALS** | **100%** | **500g** |

The compositions of the Examples were compared to demonstrate the ability of the compositions of the present invention to reduce malodour when in contact with human hair as compared with the composition of the Comparative Example.

Each sample was mixed with finely cut human hair of Asian origin in separate petri dishes at a ratio of 1:10 (Hair to Cream) and placed inside separate 1200cm³ bell jars. These were then sealed using vacuum grease, and a stopper, and labelled accordingly with coded numbers. The 1:10 ratio equated to each bell jar containing 0.5g of hair to 5g of test cream.

The bell jars were then left for a period of 15 minutes to allow for any malodour generation to collect in the headspace of the bell jar.

Volunteers were asked to remove the stopper, and first smell the reference sample which was identical to the composition of Comparative Example 1 - which they were asked to assign the number 10 for level of malodour. The volunteers were then asked to smell the three test samples and mark each sample on the scale provided for presence of malodour

The test sheets randomised the order in which the samples were tested. This randomisation was done using a Latin square technique.

Two repetitions were carried out. Each repetition involved 30 volunteers taking a sniff-test of the reference sample and then the three test samples, filling out the score sheet accordingly as they progressed. For each repetition fresh samples were made.

The scale the volunteers were presented with was from 0 to 10 with the two following references:
0 - No Malodour
10 - Malodour at the same level as the reference sample.

### Repetition One:

**Table 1**

| S**ample** | **Volunteer Size** | **Mean Malodour Score given by volunteer** | **Result Group** |
|---|---|---|---|
| Comparative Example 1 | 30 | 8.20 | A |
| Example 1 | 30 | 2.80 | B |
| Example 2 | 30 | 2.73 | B |

| | | | |
|---|---|---|---|
| L.S.D: 1.05 | | | |

### Repetition Two:

**Table 2**

| **Sample** | **Volunteer Size** | **Mean Malodour Score given by volunteer** | **Result Group** |
|---|---|---|---|
| Comparative Example 1 | 30 | 7.00 | A |
| Example 1 | 30 | 2.87 | B |
| Example 2 | 30 | 2.77 | B |

| | | | |
|---|---|---|---|
| L.S.D: 1.05 | | | |

The results presented were analysed using analysis of variance and L.S.D. at 5% to identify significant differences.

The difference between the malodour levels with the same letter is not significant. However the two result groups assigned different letters have a significant difference between them.

Both repetition results show that there is a statistically significant reduction in malodour generation between samples containing TBHQ at both the percentages of 0.1% and 0.05% compared with the standard sample containing no TBHQ.

### Example 3

A depilatory composition comprising 0.09 wt% TBHQ was prepared similarly as in Example 1. The composition was as follows:

| **Ingredient (Chemical name)** | **% w/w** |
|---|---|
| | |
| Cetearyl Alcohol | 1.5 |
| Ceteareth-20 | 0.2 |
| petrolatum Codex | 0.5 |
| paraffin wax | 0.5 |
| Shea Butter | 0.5 |
| | |
| Deionised Water | 20 |
| | |
| Deionised Water | 30.5 |
| NaOH 47% | 5.6 |
| Carbomer | 0.5 |
| Urea | 8 |
| Na Silicate | 3 |
| potassium Thioglycolate 30% | 15 |
| | |
| Deionised Water | 13.81 |
| | |
| TBHQ | 0.09 |
| propylene Glycol | 0.3 |
| | |
| **TOTAL** | **100%** |

The depilatory composition resulted in a reduction of bad odour when compared to depilatory compositions which do not contain TBHQ.

### Example 4

A Standard Base Formulation depilatory composition not containing any quinone or phenol compound was prepared similarly as in Example 1. The composition was as follows:

| **Ingredient ( Trade Name )** | **% w/w** | **100g Lab Sample (g)** |
|---|---|---|
| Deionised Water | 45.895 | 45.895 |
| Calcium Hydroxide | 3.75 | 3.75 |
| Sodium Gluconate | 0.1 | 0.1 |
| Magnesium Trisilicate | 0.5 | 0.5 |
| | | |
| Ceteareth-20 | 1.43 | 1.43 |
| Cetearyl alcohol | 3.6 | 3.6 |
| PPG-15 Stearyl Ether | 1 | 1 |
| Thick Mineral Oil | 0.1 | 0.1 |
| Sweet Almond Oil | 0.1 | 0.1 |
| | | |
| Deionised Water | 21.325 | 21.325 |
| Urea | 8 | 8 |
| Lithium Magnesium Sodium Silicate | 0.2 | 0.2 |
| Acrylates Copolymer | 0.1 | 0.1 |
| | | |
| Potassium thioglycolate | 12.9 | 12.9 |
| | | |
| Potassium Hydroxide ( 50% Solution) | 1 | 1 |
| | | |
| **TOTALS** | **100%** | **100g** |

Five further comparative depilatory compositions were prepared similarly as in Example 1. Each composition contained 1% 2 Keto-L-Gulonic Acid (In Water), 1% Pyrogallol (In Propylene Glycol), 1% Hydroquinone (In Propylene Glycol), 0.05% Hydroquinone (In Propylene Glycol), 0.05% TBHQ(In Propylene Glycol). The compositions were as follows:

**1% 2 Ke to-L-Gulonic Acid (In Water)**

| **Ingredient ( Trade Name )** | **% w/w** | **100g Lab Sample (g)** |
|---|---|---|
| Deionised Water | 38.895 | 38.895 |
| Calcium Hydroxide | 3.75 | 3.75 |
| Sodium Gluconate | 0.1 | 0.1 |
| Magnesium Trisilicate | 0.5 | 0.5 |
| | | |
| Ceteareth-20 | 1.43 | 1.43 |
| Cetearyl alcohol | 3.6 | 3.6 |
| PPG-15 Stearyl Ether | 1 | 1 |
| Thick Mineral Oil | 0.1 | 0.1 |
| Sweet Almond Oil | 0.1 | 0.1 |
| | | |
| Deionised Water | 21.325 | 21.325 |
| Urea | 8 | 8 |
| Lithium Magnesium Sodium Silicate | 0.2 | 0.2 |
| Acrylates Copolymer | 0.1 | 0.1 |
| | | |
| Potassium thioglycolate | 12.9 | 12.9 |
| | | |
| Potassium Hydroxide ( 50% Solution) | 1 | 1 |
| | | |
| 2 Keto-L-Gulonic Acid | 1 | 1 |
| Deionised Water | 6 | 6 |
| | | |
| **TOTALS** | **100%** | **100g** |

**1% pyrogallol (In Propylene Glycol)**

| **Ingredient ( Trade Name )** | **% w/w** | **100g Lab Sample (g)** |
|---|---|---|
| Deionised Water | 38.895 | 38.895 |
| Calcium Hydroxide | 3.75 | 3.75 |
| Sodium Gluconate | 0.1 | 0.1 |
| Magnesium Trisilicate | 0.5 | 0.5 |
| | | |
| Ceteareth-20 | 1.43 | 1.43 |
| Cetearyl alcohol | 3.6 | 3.6 |
| PPG-15 Stearyl Ether | 1 | 1 |
| Thick Mineral Oil | 0.1 | 0.1 |
| Sweet Almond Oil | 0.1 | 0.1 |
| | | |
| Deionised Water | 21.325 | 21.325 |
| Urea | 8 | 8 |
| Lithium Magnesium Sodium Silicate | 0.2 | 0.2 |
| Acrylates Copolymer | 0.1 | 0.1 |
| | | |
| Potassium thioglycolate | 12.9 | 12.9 |
| | | |
| Potassium Hydroxide ( 50% Solution) | 1 | 1 |
| | | |
| Pyrogallol | 1 | 1 |
| Propylene Glycol | 6 | 6 |
| | | |
| **TOTALS** | **100%** | **100g** |

**1% Hydroquinone (In Propylene Glycol)**

| **Ingredient ( Trade Name )** | **% w/w** | **100g Lab Sample (g)** |
|---|---|---|
| Deionised Water | 38.895 | 38.895 |
| Calcium Hydroxide | 3.75 | 3.75 |
| Sodium Gluconate | 0.1 | 0.1 |
| Magnesium Trisilicate | 0.5 | 0.5 |
| | | |
| Ceteareth-20 | 1.43 | 1.43 |
| Cetearyl alcohol | 3.6 | 3.6 |
| PPG-15 Stearyl Ether | 1 | 1 |
| Thick Mineral Oil | 0.1 | 0.1 |
| Sweet Almond Oil | 0.1 | 0.1 |
| | | |
| Deionised Water | 21.325 | 21.325 |
| Urea | 8 | 8 |
| Lithium Magnesium Sodium Silicate | 0.2 | 0.2 |
| Acrylates Copolymer | 0.1 | 0.1 |
| | | |
| Potassium thioglycolate | 12.9 | 12.9 |
| | | |
| Potassium Hydroxide ( 50% Solution) | 1 | 1 |
| | | |
| Hydroquinone | 1 | 1 |
| Propylene Glycol | 6 | 6 |
| | | |
| **TOTALS** | **100%** | **100g** |

**0.05% Hydroquinone (In Propylene Glycol)**

| **Ingredient ( Trade Name)** | **% w/w** | **100g Lab Sample (g)** |
|---|---|---|
| Deionised Water | 39.845 | 39.845 |
| Calcium Hydroxide | 3.75 | 3.75 |
| Sodium Gluconate | 0.1 | 0.1 |
| Magnesium Trisilicate | 0.5 | 0.5 |
| | | |
| Ceteareth-20 | 1.43 | 1.43 |
| Cetearyl alcohol | 3.6 | 3.6 |
| PPG-15 Stearyl Ether | 1 | 1 |
| Thick Mineral Oil | 0.1 | 0.1 |
| Sweet Almond Oil | 0.1 | 0.1 |
| | | |
| Deionised Water | 21.325 | 21.325 |
| Urea | 8 | 8 |
| Lithium Magnesium Sodium Silicate | 0.2 | 0.2 |
| Acrylates Copolymer | 0.1 | 0.1 |
| | | |
| potassium thioglycolate | 12.9 | 12.9 |
| | | |
| Potassium Hydroxide ( 50% Solution) | 1 | 1 |
| | | |
| 0.05% Hydroquinone | 0.05 | 0.05 |
| propylene Glycol | 6 | 6 |
| | | |
| **TOTALS** | **100%** | **100g** |

**0.05% TBHQ(In Propylene Glycol)**

| **Ingredient ( Trade Name )** | **% w/w** | **100g Lab Sample (g)** |
|---|---|---|
| Deionised Water | 45.545 | 45.545 |
| Calcium Hydroxide | 3.75 | 3.75 |
| Sodium Gluconate | 0.1 | 0.1 |
| Magnesium Trisilicate | 0.5 | 0.5 |
| | | |
| Ceteareth-20 | 1.43 | 1.43 |
| Cetearyl alcohol | 3.6 | 3.6 |
| PPG-15 Stearyl Ether | 1 | 1 |
| Thick Mineral Oil | 0.1 | 0.1 |
| Sweet Almond Oil | 0.1 | 0.1 |
| | | |
| Deionised Water | 21.325 | 21.325 |
| Urea | 8 | 8 |
| Lithium Magnesium Sodium Silicate | 0.2 | 0.2 |
| Acrylates Copolymer | 0.1 | 0.1 |
| | | |
| Potassium thioglycolate | 12.9 | 12.9 |
| | | |
| Potassium Hydroxide ( 50% Solution) | 1 | 1 |
| | | |
| 0.05% TBHQ | 0.05 | 0.05 |
| Propylene Glycol | 0.3 | 0.3 |
| | | |
| **TOTALS** | **100%** | **100g** |

The compositions of the examples were compared to demonstrate the ability of the compositions of the present invention to reduce malodour when in contact with human hair as compared with the composition of the standard base formulation. Malodour tests were carried out similarly to the tests in Example 2.

### Results:

| | Results | | |
|---|---|---|---|
| Ingredient | Volunteer Size | Mean Score | Result Group |
| Blind Standard (Standard Formulation) | 27 | 7.89 | A |
| 2-Keto-L-Gulonic Acid (Present at 1% w/w) | 27 | 7.63 | AB |
| Hydroquinone(Presen t at 1% w/w) | 27 | 6.52 | BC |
| Hydroquinone(Presen t at 0.05% w/w) | 27 | 6.00 | C |
| Pyrogallol(Present at 1% w/w) | 27 | 5.63 | C |
| TBHQ (Present at 0.05% w/w) | 27 | 3.93 | D |

The results indicate that there is a statistically significant reduction in malodour generation between depilatory compounds that contain quinone or a phenol compound than depilatory compounds that do not.

## Claims

1. A depilatory composition comprising a depilatory compound having a thiol group, and t-butyl hydroquinone.

2. A composition according to claim 1 wherein the depilatory compound is potassium thioglycolate, dithioerythritol, thioglycerol, thioglycol, thioxanthine, thiosalicylic acid, N-acetyl-L-cysteine, lipoic acid, sodium dihydrolipoate 6,8-dithioocatanoate, sodium 6,8-diothioocatanoate, a hydrogen sulphide salt, thioglycolic acid, 2-mercaptopropionic acid, 3-mercaptopropionic acid, thiomalic acid, ammonium thioglycolate, glyceryl monothioglycolate, monoethanolamine thioglycolate, monoethanolamine thioglycolic acid, diammoniumdithiodiglycolate, ammonium thiolactate, monoethanolamine thiolactate, thioglycolamide, homocysteine, cysteine, glutathione, dithiothreitol, dihydrolipoic acid, 1,3-dithiopropanol, thioglycolamide, glycerylmonothioglycolate, thioglycolhydrazine, keratinase, guanidine thioglycolate, calcium thioglycolate and/or cysteamine.

3. A composition according to claim 2 wherein the depilatory compound is potassium thioglycolate.

4. A composition according to any one of the preceding claims which comprises from 1 to 10 wt% of the depilatory compound based on the total weight of the composition.

5. A composition according to any one of the preceding claims which comprises from 0.001 to 10 wt% of the t-butyl hydroquinone based on the total weight of the composition.

6. A composition according to any one of the preceding, claims having a pH of at least 12.

7. A method of depilation comprising:
a. applying to the skin a composition as defined in any one of the preceding claims;
b. allowing the composition a residence time on the skin to degrade hairs;
c. at the end of the residence time removing the composition and depilated hairs from the skin; and
d. rinsing the skin.

8. Use of t-butyl hydroquinone in a depilatory composition comprising a depilatory compound having a thiol group to reduce the malodour of said depilatory compound when said composition is in use.

9. A method of depilation comprising
a. providing a composition as defined in any one of claims 1 to 6 in a container,
b. applying the composition to the skin such that the composition is exposed to the air,
c. allowing the composition to reside on the skin exposed to air until a colour change of the composition occurs,
d. removing the composition and depilated hairs from the skin after the colour change of the composition has occurred, and
e. rinsing the skin.

10. Use of t-bUtyl hydroquinone in a depilatory composition comprising a depilatory compound having a thiol group to provide a.colour change to said composition after said composition has been exposed to the air on the skin of a user for a predetermined time.

11. Use of t-butyl hydroquinone in combination with a monovalent and/or a divalent hydroxide in a depilatory composition comprising a depilatory compound having a thiol group, to provide a colour change to the composition after the composition has been exposed to the air whilst on the skin of a user for a predetermined time.

## Patentansprüche

1. Enthaarungszusammensetzung, umfassend eine Enthaarungsverbindung mit einer Thiolgruppe und t-Butylhydrochinon.

2. Zusammensetzung nach Anspruch 1, wobei es sich bei der Enthaarungsverbindung um Kaliumthioglycolat, Dithioerythritol, Thioglycerol, Thioglycol, Thioxanthin, Thiosalicylsäure, N-Acetyl-L-cystein, Lipoinsäure, Natriumdihydrolipoat, 6,8-Dithioocatanoat, Natrium-6,8-diothiooctanoat, ein Hydrogensulfidsalz, Thioglycolsäure, 2-Mercaptopropionsäure, 3-Mercaptopropionsäure, Thioäpfelsäure, Ammoniumthioglycolat, Glycerylmonothioglycolat, Monoethanolaminthioglycolat, Monoethanolaminthioglycolsäure, Diammoniumdithiodiglycolat, Ammoniumthiolactat, Monoethanolaminthiolactat, Thioclycolamid, Homocystein, Cystein, Glutathion, Dithiothreitol, Dihydrolipoinsäure, 1,3-Dithiopropanol, Thioglycolamid, Glycerylmonothioglycolat, Thioglycolhydrazin, Keratinase, Guanidinthioglycolat, Calciumthioglycolat und/oder Cysteamin handelt.

3. Zusammensetzung nach Anspruch 2, wobei es sich bei der Enthaarungsverbindung um Kaliumthioglycolat handelt.

4. Zusammensetzung nach einem der vorangehenden Ansprüche, die 1 bis 10 Gew.-% der Enthaarungsverbindung auf der Basis des Gesamtgewichts der Zusammensetzung umfasst.

5. Zusammensetzung nach einem der vorangehenden Ansprüche, die 0,001 bis 10 Gew.-% des t-Butylhydrochinons auf der Basis des Gesamtgewichts der Zusammensetzung umfasst.

6. Zusammensetzung nach einem der vorangehenden Ansprüche mit einem pH-Wert von mindestens 12.

7. Enthaarungsverfahren, umfassend:
a. Auftragen einer wie in einem der vorangehenden Ansprüche definierten Zusammensetzung auf die Haut;
b. Belassen der Zusammensetzung für eine Verweilzeit auf der Haut, um Haare abzubauen;
c. Entfernen der Zusammensetzung und von entfernten Haaren von der Haut am Ende der Verweilzeit; und
d. Abspülen der Haut.

8. Verwendung von t-Butylhydrochinon in einer Enthaarungszusammensetzung, die eine Enthaarungsverbindung mit einer Thiolgruppe umfasst, zum Reduzieren des üblen Geruchs der Enthaarungsverbindung, bei Verwendung der Zusammensetzung.

9. Enthaarungsverfahren, umfassend:
a. Bereitstellen einer wie in einem der Ansprüche 1 bis 6 definierten Zusammensetzung in einem Behälter;
b. Auftragen der Zusammensetzung auf die Haut derart, dass die Zusammensetzung Luft ausgesetzt wird;
c. Verweilenlassen der Zusammensetzung auf der Haut, indem sie Luft ausgesetzt wird, bis eine Farbänderung der Zusammensetzung erfolgt;
d. Entfernen der Zusammensetzung und von entfernten Haaren von der Haut nach Erfolgen der Farbänderung der Zusammensetzung; und
e. Abspülen der Haut.

10. Verwendung von t-Butylhydrochinon in einer Enthaarungszusammensetzung, die eine Enthaarungsverbindung mit einer Thiolgruppe umfasst, zum Bereitstellen einer Farbänderung der Zusammensetzung, nachdem die Zusammensetzung auf der Haut eines Anwenders für eine vorbestimmte Zeitdauer Luft ausgesetzt wurde.

11. Verwendung von t-Butylhydrochinon in Kombination mit einem einwertigen und/oder zweiwertigen Hydroxid in einer Enthaarungszusammensetzung, die eine Enthaarungsverbindung mit einer Thiolgruppe umfasst, zum Bereitstellen einer Farbänderung der Zusammensetzung, nachdem die Zusammensetzung Luft ausgesetzt wurde, während sie auf der Haut eines Anwenders für eine vorbestimmte Zeitdauer vorlag.

## Revendications

1. Composition épilatoire comprenant un composé épilatoire comprenant un groupe thiol, et de la tertiobutyl-hydroquinone.

2. Composition suivant la revendication 1, dans laquelle le composé épilatoire est le thioglycolate de potassium, le dithioérythritol, le thioglycérol, le thioglycol, la thioxanthine, l'acide thiosalicylique, la N-acétyl-L-cystéine, l'acide lipoïque, le 6,8-dithiooctanoate de dihydrolipoate de sodium, le 6,8-dithiooctanoate de sodium, un sel d'hydrogène sulfuré, l'acide thioglycolique, l'acide 2-mercaptopropionique, l'acide 3-mercaptopropionique, l'acide thiomalique, le thioglycolate d'ammonium, le monothioglycolate de glycéryle, le thioglycolate de monoéthanolamine, l'acide monoéthanolamine-thioglycolique, le dithioglycolate de diammonium, le thiolactate d'ammonium, le thiolactate de monoéthanolamine, le thioglycolamide, l'homocystéine, la cystéine, le glutathion, le dithioérythritol, l'acide dihydrolipoïque, le 1,3-dithiopropanol, le thioglycolamide, le monothioglycolate de glycéryle, la thioglycolhydrazine, la kératinase, le thioglycolate de guanidine, le thioglycolate de calcium et/ou la cystéamine.

3. Composition suivant la revendication 2, dans laquelle le composé épilatoire est le thioglycolate de potassium.

4. Composition suivant l'une quelconque des revendications précédentes, qui comprend 1 à 10 % en poids du composé épilatoire sur la base du poids total de la composition.

5. Composition suivant l'une quelconque des revendications précédentes, qui comprend 0,001 à 10 % en poids de la tertiobutyl-hydroquinone sur la base du poids total de la composition.

6. Composition suivant l'une quelconque des revendications précédentes, ayant un pH d'au moins 12.

7. Procédé d'épilation, comprenant les étapes consistant à :
a. appliquer à la peau une composition telle que définie dans l'une quelconque des revendications précédentes ;
b. laisser la composition pendant un temps de séjour sur la peau pour la dégradation des poils ;
c. à la fin du temps de séjour, éliminer la composition et les poils épilés de la peau ; et
d. rincer la peau.

8. Utilisation de tertiobutyl-hydroquinone dans une composition épilatoire comprenant un composé épilatoire comprenant un groupe thiol pour réduire l'odeur désagréable dudit composé épilatoire lors de l'utilisation de ladite composition.

9. Procédé d'épilation, comprenant les étapes consistant à :
a. fournir une composition telle que définie dans l'une quelconque des revendications 1 à 6 dans un récipient ;
b. appliquer la composition à la peau de telle sorte que la composition soit exposée à l'air ;
c. laisser la composition résider sur la peau exposée à l'air jusqu'à ce qu'un changement de couleur de la composition se produise ;
d. éliminer la composition et les poils épilés de la peau après que le changement de couleur de la composition s'est produit ; et
e. rincer la peau.

10. Utilisation de tertiobutyl-hydroquinone dans une composition épilatoire comprenant un composé épilatoire ayant un groupe thiol pour provoquer un changement de couleur de ladite composition après exposition de ladite composition à l'air sur la peau d'un utilisateur pendant une période de temps prédéterminée.

11. Utilisation de tertiobutyl-hydroquinone en association avec un hydroxyde monovalent et/ou divalent dans une composition épilatoire comprenant un composé épilatoire ayant un groupe thiol, pour provoquer un changement de couleur de la composition après exposition de la composition à l'air tandis que la composition est présente sur la peau d'un utilisateur pendant une période de temps prédéterminée.
